# EUROPEAN PATENT APPLICATION

(11) **EP 1 557 146 A1**
(43) Date of publication of application: **27.07.2005**
(21) Application number: 03742861.2
(22) Date of filing: 09.04.2003
(51) Int. Cl.: A61F 7/08, A61K 9/70

(54) **HEAT GENERATING ARTICLE**

(30) Priority: 31.10.2002 JP 2002318000
(71) Applicant: Mycoal Products Corporation, Tochigi-chi, Tochigi 328-0067 (JP)
(72) Inventor: USUI, Kaoru c/o Mycoal Warmers Co., Ltd., Tochigi-shi, Tochigi 328-0067 (JP); KURIHARA, Makoto c/o Mycoal Warmers Co., Ltd., Tochigi-shi, Tochigi 328-0067 (JP); URUME, Yukio c/o Mycoal Warmers Co., Ltd., Tochigi-shi, Tochigi 328-0067 (JP); OMAE, Hirotaka c/o Mycoal Warmers Co., Ltd., Tochigi-shi, Tochigi 328-0067 (JP)
(74) Representative: Körfer, Thomas, Dipl.-Phys.
(86) International application number: PCT/JP2003/004492
(87) International publication number: WO 2004/039294

(57) **Abstract**

The present invention relates to a heat-generating body containing a heat-generating composition causing a heat-generating reaction in the presence of air sealed in an air permeable container body having a desired shape, such as a bag form, a sheet form and the like, and a cohesive layer provided.

The invention aims to such an object that chill feeling to a human body upon application and in the initial service period, which is a problem of a water-containing hydrophilic gel, is reduced to provide a heat-generating body suitable for using as a heat-generating sheet agent or the like having a water-soluble hydrophilic gel cohesive agent layer excellent in usability, which is a heat-generating body containing a heat-generating composition causing a heat-generating reaction in the presence of air sealed in an air permeable container body having a desired shape, such as a bag form, a sheet form and the like, and a cohesive layer containing water-containing hydrophilic gel agent obtained from a hydrophilic polymer thickener provided on the container body, **characterized in that** the water containing hydrophilic gel agent contains an organic filler.

## Description

### (Technical Field)

The present invention relates to a heat-generating body having a heat-generating composition causing a heat-generating reaction in the presence of air, sealed in an air permeable container body having a desired shape, such as a bag form, a sheet form and the like, and a cohesive agent layer provided therewith. More specifically, it relates to a heat-generating body that is reduced in chill feeling to a human body upon direct attachment to skin, and is preferred for using as a heat-generating sheet agent for attaching on skin being excellent in usability.

### (Background Art)

A heat-generating body used as a heat-generating sheet agent or the like generally contains a heat-generating body part constituted by sealing a heat-generating composition containing an iron compound in an air permeable container body in a bag form, a sheet form or the like, and a cohesive layer part provided on one surface thereof for adhering on skin. As the cohesive layer of the heat-generating sheet agent, a large number of products using a nonaqueous cohesive agent have been proposed.

However, because the nonaqueous cohesive agent does not absorb moisture, body fluids, such as sweat and the like, are retained between the surface of skin and the cohesive layer upon attachment on skin due to heat of the heat-generating body, and thus it is liable to be released due to reduction of the cohesive force of the cohesive layer. In the case where a strong cohesive force is provided, on the other hand, such problems are caused that releasing is associated with pain due to breakage of cuticle on the surface of skin and pulling of skin hair.

On the other hand, a heat-generating body used as a heat-generating sheet agent or the like using a hydrophilic cohesive agent uses a water-containing hydrophilic gel, which is generally used in wet compress and the like, whereby there is a problem of coldness (chill feeling) to a human body due to water in the gel upon attachment and in the initial service period, and in particular, it is serious to a user in the case where it is used in cold season in winter. As a material for preventing chill feeling in the initial attachment period for a wet compress, JP-A-8-268879 proposes a material having a tactile sense relaxing layer that relaxes tactile sense (coldness) of a wet compress by intervening between a wet compress base agent layer and an affected area upon attachment. According to the proposal, upon applying a wet compress on an affected area, the tactile sense relaxing layer is firstly in contact with the affected area, and the tactile sense relaxing layer is buried in the wet compress base agent when the wet compress base agent layer is heated and softened with heat conduction and radiant heat, whereby the wet compress base agent is in contact with skin in such a state that the temperature thereof is close to the skin temperature. It is said that, according to the configuration, upon attaching the wet compress, the tactile (coldness) of the wet compress base agent is not directly transmitted to skin of the affected area but is relaxed. However, the weight of the total wet compress is necessarily retained only with the cohesive force of the skin contact part of the tactile sense relaxing layer, and it is not practical measure. Furthermore, the tactile sense relaxing layer has a thickness that is buried on the skin with the lapse of time, and therefore, it causes such a problem that the wet compress base agent is in contact with the skin to impart chill feeling depending on differences in elasticity of skin caused by stiffness of skin with respect to individual difference or difference in position. There is no another proposal for lowering the chill feeling, and it is the current situation that the problem has not yet been resolved.

However, a water-containing hydrophilic gel has such characteristics that moisture, such as sweat and the like, secreted from a body is absorbed, releasing is associated with no pain since breakage of cuticle on the surface of skin and pulling of skin hair do not occur, and a water soluble substance, such as an antiallergenic substance, a moisture retaining agent, a percutaneous absorption agent and the like, can be contained in the water-containing hydrophilic gel, whereby it is mild and safe to skin in comparison to the nonaqueous cohesive agent.

An object of the invention is to provide a heat-generating body that is reduced in chill feeling to a human body upon application and in the initial service period, which is the problemof the water-containing hydrophilic gel, and is preferred for using as a heat-generating sheet agent having the water-containing hydrophillic for attaching on skin being excellent in usability.

### (Disclosure of the Invention).

As a result of earnest investigations made by the inventors for attaining the obj ect, it has been found that a heat-generating sheet agent that is reduced in chill feeling to a human body upon application and the initial service period and is excellent in usability can be obtained in such a manner that, in a heat-generating body obtained by sealing a heat-generating composition causing a heat-generating reaction in the presence of air, in an air permeable container body having a desired shape, such as a bag form, a sheet form and the like, and providing therewith a cohesive agent layer containing a water-containing hydrophilic gel agent obtained from a hydrophilic polymer thickener, a layer containing a hydrophilic gel agent containing an organic filler as an essential component is provided, and thus the invention has been completed.

That is, the heat-generating body of the invention is, as described in the claim 1, a heat-generating body containing a heat-generating composition causing a heat-generating reaction in the presence of air sealed in an air permeable container body having a desired shape, such as a bag form, a sheet form and the like, and a cohesive layer containing water-containing hydrophilic gel agent obtained from a hydrophilic polymer thickener provided on the container body, characterized in that the water contain hydrophilic gel agent contains an organic filler.

A heat-generating body of claim 2 is a heat-generating body as described in claim 1, characterized in that the organic filler is porous.

A heat-generating body of claim 3 is a heat-generating body as described in claim 1, characterized in that the organic filler is hydrophilic.

A heat-generating body of claim 4 is a heat-generating body as described in claim 1, characterized in that the organic filler is at least one kind selected from crystalline cellulose, wood powder, vegetable dry powder, pulp, regenerated cellulose and fiber chips.

A heat-generating body of claim 5 is a heat-generating body as described in claims 1 to 4, characterized in that the hydrophilic polymer thickener is at least one kind selected from polyacrylic acid, a polyacrylate salt and a cellulose derivative.

A heat-generating body of claim 6 is a heat-generating body as described in claims 1 to 4, characterized in that the water-containing hydrophilic gel agent contains from 1 to 30% by weight of a hydrophilic polymer thickener, from 1 to 80% by weight of water, 80% by weight or less of a moisture retaining agent, 5% by weight or less of a curing agent, and from 1 to 30% by weight of an organic filler.

A heat-generating body of claim 7 is a heat-generating body as described in claim 6, characterized in that the moisture retaining agent is at least one kind selected from a polyhydric alcohol and a saccharide.

A heat-generating body of claim 8 is a heat-generating body as described in claim 6, characterized in that the curing agent is at least one kind selected from a divalent metallic compound and a trivalent metallic compound.

A heat-generating body of claim 9 is a heat-generating body as described in claims 1 to 4, characterized in that the heat-generating body is a heat-generating sheet agent for attaching on skin.

### (Brief Description of Drawing)

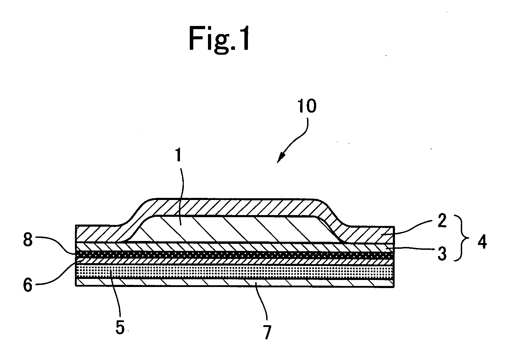

Fig. 1 shows an explanatory cross sectional view of one example of a heat-generating body (heat-generating sheet agent) of the invention.

### (Best Mode for carrying out the Invention)

Embodiments of the invention will be described in detail below.

In the heat-generating body of the invention, as described in the foregoing, an organic filler is contained in a water-containing hydrophilic gel agent, whereby chill feeling to a human body upon application and in the initial service period is reduced to provide a heat-generating body excellent in usability.

The water-containing hydrophilic gel agent used in the heat-generating body of the invention is preferably constituted with, as major components, from 1 to 30% by weight of at least one kind of a hydrophilic polymer thickener component selected, for example, from polymer thickeners, such as polyacrylic acid, a polyacrylate salt, a cellulose derivative and the like, from 1 to 80% by weight of water, from 0 to 80% by weight of a moisture retaining agent, from 0 to 5% by weight of a curing agent, such as a divalent metallic compound and/or a trivalent metallic compound and the like, from 1 to 30% by weight of an organic filler, from the standpoint that the adhesion to skin and the shape retaining property are good.

The water-containing hydrophilic gel agent is not particularly limited and may be constituted with a hydrophilic polymer thickener, such as polyacrylic acid, a polyacrylate salt, a cellulose derivative and the like.

Any material of the polyacrylic acid may be used, and the molecular weight and the structure, e.g., linear, branched or the like, are not particularly limited.

Specific examples of the polyacrylate salt include sodium polyacrylate, potassium polyacrylate, polyacrylic acid monoethanolamine, polyacrylic acid diethanolamine, polyacrylic acid triethanolamine, ammonium polyacrylate and the like.

Specific examples of the cellulose derivative include carboxymethyl cellulose, sodium carboxymethyl cellulose, hydroxymethyl cellulose, hydroxyethyl cellulose, hydroxypropyl cellulose, hydroxypropylmethyl cellulose, hydroxypropylethyl cellulose, methyl cellulose, ethyl cellulose, ethylhydroxymethyl cellulose, cationized cellulose and the like.

In addition to the polymer thickeners, such water soluble polymer compounds are exemplified,assodium alginate,propylene glycol alginate ester, sodium starch glycolate, sodium starch phosphate ester, polyvinyl alcohol, polyvinylpyrrolidone, polyvinylpyrrolidone polyethyleneoxide, polyvinylmethylether, a carboxyvinyl polymer, polyacrylamide, polyethylene glycol, polyethyelneoxide, cyamoposis gum, gum arabic, tragant gum, gum caraya, carrageenan, agar, xanthan gum, gellan gum, curdlan, pullulan, pectin, dextrin, chitin, chitosan, chitosamine, gelatin and the like.

The mixing amount of at least one kind selected from the group consisting of the polymer thickener cohesive agents is from 1 to 30% by weight, preferably from 2 to 25% by weight, and more preferably from 3 to 15% by weight, based on the total water-containing hydrophilic gel agent composition, and in the case where the mixing amount is too small, there are some cases where the cohesive force is lowered, whereas in the case where it is too large, there are some cases where problems are caused in workability due to a high viscosity, and pain is associated with release of the sheet agent due to a large cohesive force.

The mixing amount of water used in the water-containing hydrophilic gel agent is from 1 to 80% by weight, preferably from 5 to 75% by weight, and more preferably from 10 to 70% by weight, based on the total water-containing hydrophilic gel agent composition, and in the case where the mixing amount is too small, there are some cases where a gel is not formed, whereas in the case where it is too large, there are some cases where the essential components cannot be sufficiently mixed.

With respect to the moisture retaining agent of a polyhydric alcohol and/or a saccharide, specific examples of the polyhydric alcohol include ethylene glycol, dietylene glycol, triethylene glycol, propylene ethylene glycol, dipropylene glycol, butylene glycol, hexylene glycol, glycerin, diglycerin, polyethylene glycol, polypropylene glycol, polyglycerin and the like. Specific examples of the saccharide include xylitol, sorbitol, maltitol, starch sugar and the like.

The mixing amount of the moisture retaining agent of a polyhydric alcohol and/or a saccharide with respect to the water-containing hydrophilic gel agent is from 0 to 80% by weight, preferably from 5 to 75% by weight, and more preferably from 10 to 70% by weight, based on the total water-containing hydrophilic gel agent, and in the case where it is larger than 80% by weight, the cohesive force of the gel is lowered, and there are some cases where the cohesive agent remains on skin upon releasing.

The curing agent causes crosslinking with polyacrylic acid, a polyacrylate salt, a cellulose derivative or the like, and specific examples of the divalent metallic compound include calcium hydroxide, calcium carbonate, calcium sulfate, calcium nitrate, calcium chloride, calcium acetate, calcium oxide, calcium phosphate, magnesium hydroxide, magnesium carbonate, magnesium sulfate, magnesium nitrate, magnesium chloride, magnesium silicate, magnesium oxide, magnesium alumina hydroxide, magnesium aluminometasilicate, magnesium alminosilicate, synthetic hydrotalcite and the like. Specific examples of the trivalent metallic compound include potassium alum, ammonium alum, ferric alum, aluminum hydroxide, aluminum sulfate, aluminum chloride, aluminum aluminoglycinatoacetate, aluminum oxide, silicic acid-containing aluminum, aluminum metasilicate and the like.

The mixing amount of the curing agent with respect to the water-containing hydrophilic gel agent is from 0 to 5% by weight, preferably from 0.01 to 3% by weight, and more preferably from 0.1 to 2% by weight, based on the total water-containing hydrophilic gel agent, and in the case where it is more than 5% by weight, there are some cases where problems occur in workability, and the gel becomes stiff to lower the cohesive force, whereby a suitable cohesive force cannot be obtained.

The organic filler used in the water-containing hydrophilic gel agent is an important component in the invention, and it lowers chill feeling to a human body upon direct application of the heat-generating body to skin and in the initial service period. In general, in the case where a filler is used in a wet compress, an application agent, a cosmetic gel sheet or the like, it is used for such purposes that the viscosity of the cohesive layer is adjusted, it functions as a crosslinking agent to improve shape retaining property, and it functions as an extender. Furthermore, most of them are inorganic filler.

In the invention, an organic filler is mixed in the water-containing hydrophilic gel agent, whereby the low heat conduction and the low heat capacity of the organic filler are utilized with the functions of the filler being maintained, and as a result, the chill feeling to a human body upon direct application of the heat-generating sheet agent to skin and in the initial service period can be lowered.

In more detail, it has been found that in a nonsteady state where the temperature of a human body and the temperature of the heat-generating sheet agent are not sufficiently raised, the temperature increasing rate per unit period of time on an interface in contact with skin is not increased owing to the low heat conduction and the low heat capacity, and thus the body temperature of the human body is not quickly lowered to suppress the chill feeling.

The mixing amount of the organic filler with respect to the water-containing hydrophilic gel agent is from 1 to 30% by weight, preferably from 5 to 25% by weight, and more preferably from 10 to 20% by weight, based on the total water-containing hydrophilic gel agent, and in the case where it is less than 1% by weight, no sufficient effect is obtained, whereas in the case where it is more than 30% by weight, it cannot be formed into a sheet form due to high viscosity.

The organic filler preferably has a porous particle surface since the heat conduction and the heat capacity further become lower owing to the presence of an air layer. In other words, it is more preferred to use a porous material as the organic filler since the chill feeling can be suppressed without quickly lowering the body temperature from the skin owing to the heat conduction and the low heat capacity. Moreover, in the case where the organic filler is further hydrophilic, it is preferred that the filler can be conveniently and uniformly dispersed in the water-containing hydrophilic gel agent, and thus the function of the organic filler can be effectively exerted.

Examples of the organic filler include at least one kind selected from crystalline cellulose, wood powder, vegetable dry powder, pulp, regenerated cellulose and fiber chips. In particular, crystalline cellulose is desired from the stand point of safety, handling property, storage property, stability and the like.

The crystalline cellulose is fine powder mainly containing crystals of α-cellulose having been partially treated with a mineral acid to have a low molecular weight, and examples thereof includeAvicel, atradename, produced by Asahi Kasei Corp. There are some grades depending on differences in particle size distribution of the powder and in surface treating method, and all the grades can be used.

Examples of the vegetable dry powder include linter, linen and the like. Examples of the pulp include ground wood pulp, sulfitepulp, kraft pulp, semichemical pulp, refiner ground pulp, linter pulp and the like. Examples of the regenerated cellulose include viscose rayon, cellophane and the like. Examples of the fiber chips include acetate fibers, vinylon, aramid fibers, copper ammonia fibers, silk and the like.

In the water-containing hydrophilic gel agent, such components that are generally used in a wet compress, an application agent, a cosmetic gel sheet or the like may be appropriately mixed as far as the effect of the invention is not impaired, and examples of the components include inorganic powder, a percutaneous absorption agent, an antioxidant, an antiseptic agent, a perfume, a coloring material, a coloring pigment, an emulsifier, an antiallergenic agent, a cosmetic component and the like.

The heat-generating composition used in the heat-generatingbody of the invention is not particularly limited as far as it contains components generally used in such a heat-generating body composition that is referred to as a chemical body warmer, and for example, it contains metallic powder, such as iron powder and the like, and water as major components, and also contains activated carbon having functions of causing an oxidation reaction of the metallic powder, adjusting pH and catalyzing, a chloride for breaking an oxide film on the surface of the metallic powder to attain continuous progress of the oxidation reaction, and further a water retaining agent used for removing tackiness due to water. Specific examples of the metallic powder used include cast iron powder, reduced iron powder, electrolytic iron powder and the like. Examples of the activated carbon used include palm shell carbon, charcoal powder, peat and the like. Examples of a reaction assistant used include sodium chloride, potassium chloride, magnesium chloride, calcium chloride, potassium sulfate, magnesium sulfate, sodium sulfate and the like. Examples of the water retaining agent used include vermiculite, perlite, cristobalite, silica gel, wood powder, a water absorbing polymer and the like. It is preferred that a heat-generating composition containing these specific examples has a compositional ratios of from 40 to 75% by weight of the iron powder, from 10 to 40% by weight of water and from 1 to 40% by weight of the water retaining agent, from the stand point of safety, economy, storage stability, temperature characteristics, such as initial rising, persistence period and the like, and the like.

The heat-generating body of the invention is formed by containing the heat-generating composition in an air permeable container body, and the container body is constituted, for example, with a flat packing material having air permeability and an air nonpermeable packing material in a sheet form. The flat packing material having air permeability is constituted with a base material and a covering material, and they are not particularly limited as far as such conditions are attained that at least one of them has air permeability on the whole surface of a part of at least one of them, whereby in the case where the heat-generating composition is contained, the heat-generating composition inside is not leaked, and in the case where it is constituted as a heat-generating body, such as a heat-generating sheet agent or the like, of a type of attaching to skin having the water-containing hydrophilic gel layer provided, the heat-generating composition inside generates heat owing to the air permeability to obtain a desired temperature.

The invention will be specifically described below with reference to examples and comparative examples, but the invention is not limited thereto.

50% by weight or iron powder, 5% by weight of activated carbon, 5% by weight of sodium chloride, 10% by weight of a water retaining agent and 30% by weight of water were mixed to obtain a heat-generating composition.

Thereafter, as shown in Fig. 1, it was charged in a container body 4 that was constituted by laminating an air permeable sheet 2 containing a porous film (Breathron, produced by Nitto Lifetech Corp.) formed by laminating a fine porous polyethylene sheet with a nylon spunbonded nonwoven fabric and an air nonpermeable sheet 3 formed by laminating a polyethylene film with a nylon spunbonded nonwoven fabric (produced by Asahi Kasei Corp.) into a bag form having a flat disk shape having a diameter of about 50 mm.

Separately, the components shown in Table 1 were mixed and agitated in a mixer in an ordinary procedure to prepare a water-containing hydrophilic gel agent composition. Thereafter, as shown in Fig. 1, the water-containing hydrophilic gel agent composition 5 was uniformly coated on a spun lace nonwoven fabric 6 (basis weight: 90 to 110 g/m²) of polyester fibers and polyolefin fibers lined with a polyethylene film to an amount of from 1,000 to 1,200 g/m², on which a releasing film 7 formed with casting polypropylene was overlaid, and the assembly was molded into a sheet form and packaged. After aging, it was punched out to the same size as the container body 4 having the heat-generating composition contained, and was adhered with the air nonpermeable sheet 3 of the container body 4 through a hot melt adhesive 8 to obtain a heat-generating sheet agent as a heat-generating body 10. The resulting heat-generating sheet agent was sealed in a sealed bag.

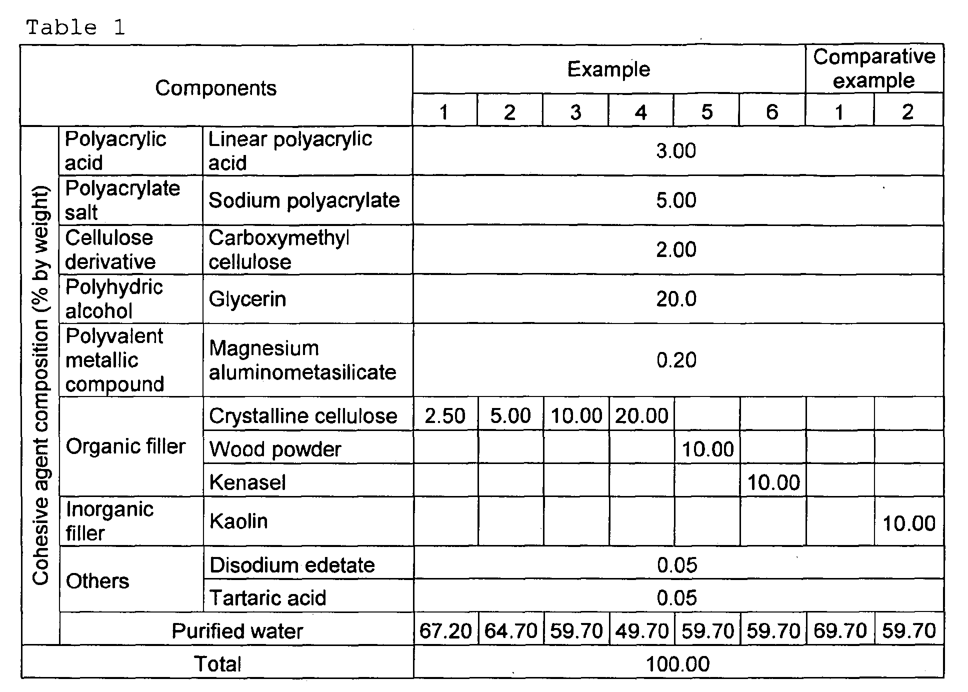

### (Evaluation of Usability)

In order to confirm the effect of the invention, the heat-generating sheet agents prepared from the components of Examples 1 to 6 and Comparative Examples 1 and 2 described in the foregoing were placed in a room set at a temperature of 20°C and a humidity of 50%, and the heat-generating sheet agents taken out from the sealed bags were immediately attached to abdominal areas of 10 expert subjects to carry out a sensory test for usability. The evaluation was standardized by the following points and expressed by an average value of the 10 subjects. The evaluation results are shown in Table 2.
2 points: Subject withstood chill feeling upon application and in initial service period.
1 point: Subject somewhat withstood chill feeling upon application and in initial service period.
0 point: Subject could not withstand chill feeling upon application and in initial service period.

**Table 2**

| | Example | | | | Comparative Example | | | |
|---|---|---|---|---|---|---|---|---|
| | 1 | 2 | 3 | 4 | 5 | 6 | 1 | 2 |
| Sensory evaluation of usability | 1.40 | 1.60 | 1.80 | 1.95 | 1.20 | 1.10 | 0.10 | 0.50 |

As found from the results in Table 2, the products of Examples were reduced in chill feeling upon application and in the initial service period in comparison to the products of Comparative Examples to provide good results.

### (Applicability in Industry)

According to the invention, a heat-generating body, such as a heat-generating sheet agent and the like, is provided that has an appropriate cohesive force, is reduced in chill feeling to a human body upon application and in the initial service period, and is excellent in usability.

## Claims

1. A heat-generating body comprising a heat-generating composition causing a heat-generating reaction in the presence of air sealed in an air permeable container body having a desired shape, such as a bag form, a sheet form and the like, and a cohesive layer containing water-containing hydrophilic gel agent obtained from a hydrophilic polymer thickener provided on the container body, **characterized in that** the water containing hydrophilic gel agent contains an organic filler.

2. A heat-generating body as described in claim 1, **characterized in that** the organic filler is porous.

3. A heat-generating body as described in claim 1, **characterized in that** the organic filler is hydrophilic.

4. A heat-generating body as described in claim 1, **characterized in that** the organic filler is at least one kind selected from crystalline cellulose, wood powder, vegetable dry powder, pulp, regenerated cellulose and fiber chips.

5. A heat-generating body as described in claims 1 to 4, **characterized in that** the hydrophilic polymer thickener is at least one kind selected from polyacrylic acid, a polyacrylate salt and a cellulose derivative.

6. A heat-generating body as described in claims 1 to 4, **characterized in that** the water-containing hydrophilic gel agent contains from 1 to 30% by weight of a hydrophilic polymer thickener, from 1 to 80% by weight of water, 80% by weight or less of a moisture retaining agent, 5% by weight or less of a curing agent, and from 1 to 30% by weight of an organic filler.

7. A heat-generating body as described in claim 6, **characterized in that** the moisture retaining agent is at least one kind selected from a polyhydric alcohol and a saccharide.

8. A heat-generating body as described in claim 6, **characterized in that** the curing agent is at least one kind selected from a divalent metallic compound and a trivalent metallic compound.

9. A heat-generating body as described in claims 1 to 4, **characterized in that** the heat-generating body is a heat-generating sheet agent for attaching on skin.
